# EUROPEAN PATENT APPLICATION

(11) **EP 2 700 417 A1**
(43) Date of publication of application: **26.02.2014**
(21) Application number: 12774387.0
(22) Date of filing: 06.04.2012
(51) Int. Cl.: A61L 9/16, A61L 9/00, B01D 53/06, B60H 3/00, F24F 7/00

(54) **AIR PURIFIER**

(30) Priority: 21.04.2011 JP 2011094875; 08.07.2011 JP 2011151364; 31.08.2011 JP 2011188764; 31.08.2011 JP 2011188765; 28.09.2011 JP 2011212404; 28.09.2011 JP 2011212405; 29.09.2011 JP 2011214646
(71) Applicant: Mitsubishi Electric Corporation, Tokyo 100-8310 (JP); Mitsubishi Electric Home Appliance Co., Ltd., Fukaya-shi, Saitama 369-1295 (JP)
(72) Inventor: YANAI, Toshiyuki, Fukaya-shi Saitama 369-1295 (JP); AKARI, Yoshitaka, Fukaya-shi Saitama 369-1295 (JP); KUGE, Yousuke, Fukaya-shi Saitama 369-1295 (JP); SATO, Akihisa, Tokyo 102-0073 (JP)
(74) Representative: Pfenning, Meinig & Partner GbR
(86) International application number: PCT/JP2012/059452
(87) International publication number: WO 2012/144342

(57) **Abstract**

An air purifier is provided that efficiently recovers a function of a deodorizing element that absorbs an odor. The air purifier includes: a body case having an inlet and an outlet, and having an air trunk that provides communication between the inlet and the outlet; blowing means that is included in the body case, and introduces indoor air through the air trunk; a deodorizing element that is provided in the air trunk, and through which the air can pass; a heating unit that locally heats the deodorizing element; a frame including the deodorizing element and the heating unit; position changing means for changing a positional relationship between the heating unit and the deodorizing element; and control means for controlling the blowing means, the heating unit, and the position changing means, the deodorizing element is heated by the heating unit to recover a deodorizing function, and is rotatably mounted to the frame, the heating unit is secured to the frame while facing the deodorizing element, and a lid covering the heating unit is provided on a facing side of the heating unit via the deodorizing element.

## Description

### Technical Field

The present invention relates to an air purifier that passes indoor air sucked into a body by a blower through a deodorizing portion to remove an odor component in the air to deodorize the indoor air.

### Background Art

Conventionally, there is an air purifier including: a body case; an air inlet formed in a front surface of the body case; an air outlet formed in a rear of an upper surface of the body case; a fan provided in the body case for sucking air from the air inlet and blowing out the air to the air outlet; a fan motor for driving the fan; and a dust collection filter provided upstream of the fan for collecting dust in the sucked air, wherein a deodorizing portion formed of a catalyst attached to a surface of an absorbent for absorbing an odor component, and a heating portion for heating to recover a deodorizing function of the deodorizing portion are provided near the air outlet.

Such an air purifier drives the fan to take indoor air from the air inlet into the body case, remove dust using the dust collection filter, and then deodorizes the indoor air by the odor component in the indoor air flowing down to the deodorizing portion being absorbed by the absorbent in the deodorizing potion.

The absorbent in the deodorizing portion having absorbed an odor is heated by the heating portion, and thus the odor component is removed therefrom, thereby recovering a deodorizing function (for example, Patent Literature 1).

### Citation List

### Patent Literature

Patent Literature 1: Japanese Patent Laid-Open No. 2001-38126 (Figure 1)

### Summary of Invention

### Problem to be Solved by the Invention

However, in the air purifier described in the Citation List, a heater that is the heating portion for heating the absorbent to remove the odor absorbed by the absorbent is secured to the absorbent, and thus there are parts with a short distance and a long distance between the heater and the absorbent.

Thus, a part of the absorbent close to the heater can be efficiently heated to remove an odor, while heat from the heater may be inefficiently transferred to a part of the absorbent remote from the heater, and there is a possibility that an odor cannot be completely removed.

Since the heater is simply provided on the absorbent, the heat from the heater easily escapes to an outside of the absorbent.

Thus, such a configuration cannot sufficiently recover an odor absorbing capacity of the absorbent, which may reduce a deodorizing function of the air purifier.

The present invention is achieved to solve the above described problems, and has an object to provide an air purifier that can efficiently recover a function of an absorbent that absorbs an odor, and can efficiently reduce an odor in indoor air.

### Means for Solving the Problem

In accomplishing the above object, there is provided an air purifier comprising: a body case having an inlet and an outlet opening outward, and having an air trunk that provides communication between the inlet and the outlet; blowing means that is included in the body case, and introduces indoor air through the air trunk from the inlet to the outlet; a deodorizing element that is provided in the air trunk, and through which the introduced air can pass; a heating unit that locally heats the deodorizing element; a frame made of synthetic resin and including the deodorizing element and the heating unit; position changing means for changing a relative positional relationship between the heating unit and the deodorizing element; and control means for controlling energization of the blowing means, the heating unit, and the position changing means, wherein the deodorizing element is heated by the heating unit to recover a deodorizing function, and is rotatably mounted to the frame, the heating unit is secured to the frame while facing the deodorizing element, and a lid covering the heating unit is provided on a facing side of the heating unit via the deodorizing element.

### Advantageous Effects of Invention

According to the present invention, an air purifier can be provided that can take in more indoor air, efficiently reduce an odor in indoor air, and can reliably efficiently recover a function of an absorbent that absorbs an odor.

### Brief Description of Drawings

Figure 1(a) is a front view showing an air purifier A according to Embodiment 1, Figure 1(b) is a top view showing the air purifier A, and Figure 1(c) is a side view showing the air purifier A.
Figure 2 is a front view (a), a top view (b) and a side view (c) showing a state in which a front panel, a prefilter and a HEPA filter were removed from an air purifier A shown in Figure 1.
Figure 3 is an exploded perspective view of an air purifier A shown in Figure 1.
Figure 4 is a Y-Y vertical cross-sectional view of an air purifier A shown in Figure 1.
Figure 5(a) is a front perspective view of an air purifier A shown in Figure 1, and Figure 5(b) is a rear perspective view of an air purifier A shown in Figure 1.
Figure 6 is a perspective view showing a state in which a front panel, a prefilter and a HEPA filter were removed from an air purifier A shown in Figure 5(a).
Figure 7(a) is a front view showing a state in which a front panel was removed from an air purifier A according to Embodiment 1, and Figure 7(b) is a Z-Z cross-sectional view of an air purifier A shown in Figure 7(a).
Figure 8(a) is a front perspective view of a deodorizing portion of an air purifier A according to Embodiment 1, and Figure 8(b) is a rear perspective view of the deodorizing portion.
Figure 9 is an exploded perspective view of a deodorizing portion of an air purifier A shown in Figure 8(b).
Figure 10 is a front perspective view of a deodorizing element according to Embodiment 1.
Figure 11 is a positional relationship between a optical sensor and a deodorizing element according to Embodiment 1.
Figure 12 is an illustration showing a X-X cross-section shown in Figure 11(a).
Figure 13(a) is a plan view showing a back surface of a heating unit of an air purifier A according to Embodiment 1, and Figure 13(b) is a Z-Z cross-sectional view of the heating unit.
Figure 14(a) is a perspective view showing a back surface of a heating unit shown in Figure 10, and Figure 14(b) is a perspective view showing a front surface of a heating unit shown in Figure 10.
Figure 15 is a front view (a), a top view (b) and a side view (c) showing an air purifier A according to Embodiment 1.
Figure 16(a) is a front perspective view of an air purifier A shown in Figure 15, and Figure 16(b) is a rear perspective view of the air purifier A.
Figure 17 is a bottom view of an air purifier shown in Figure 15.

### Modes for Carrying Out the Invention

### (Embodiment 1)

Now, Embodiment 1 of the present invention will be described with reference to the drawings.

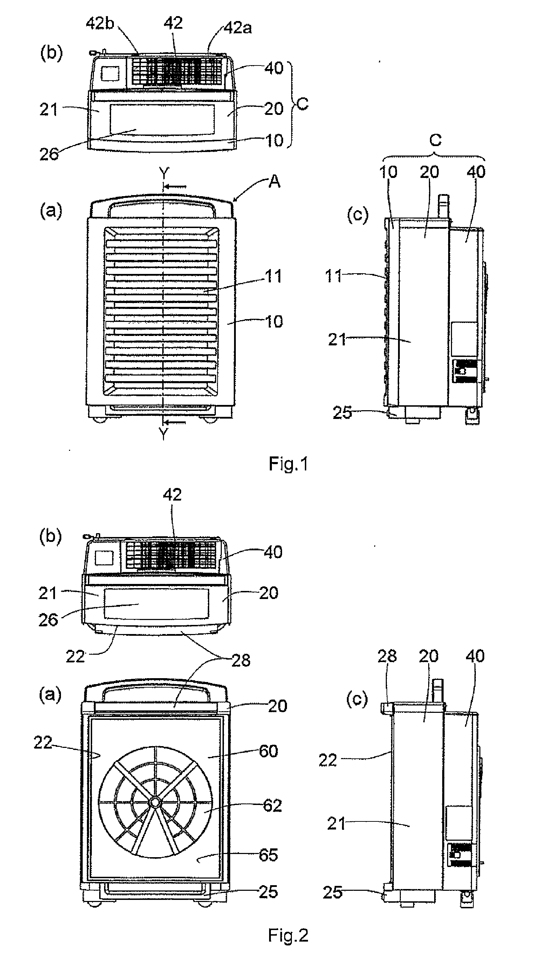

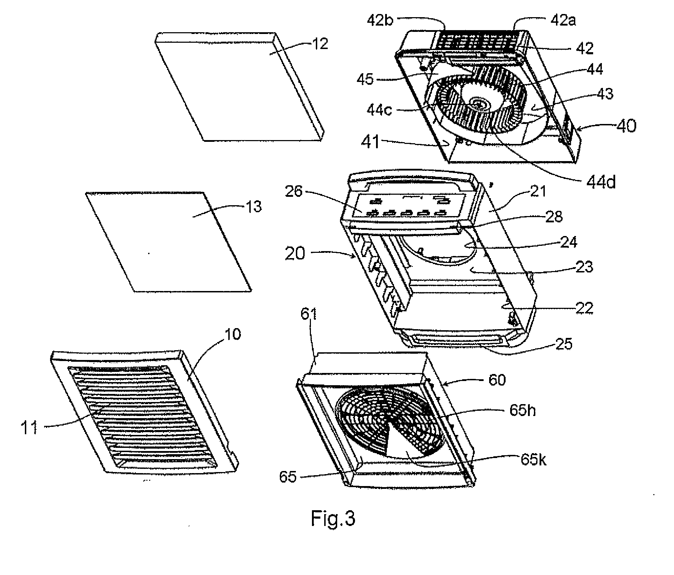

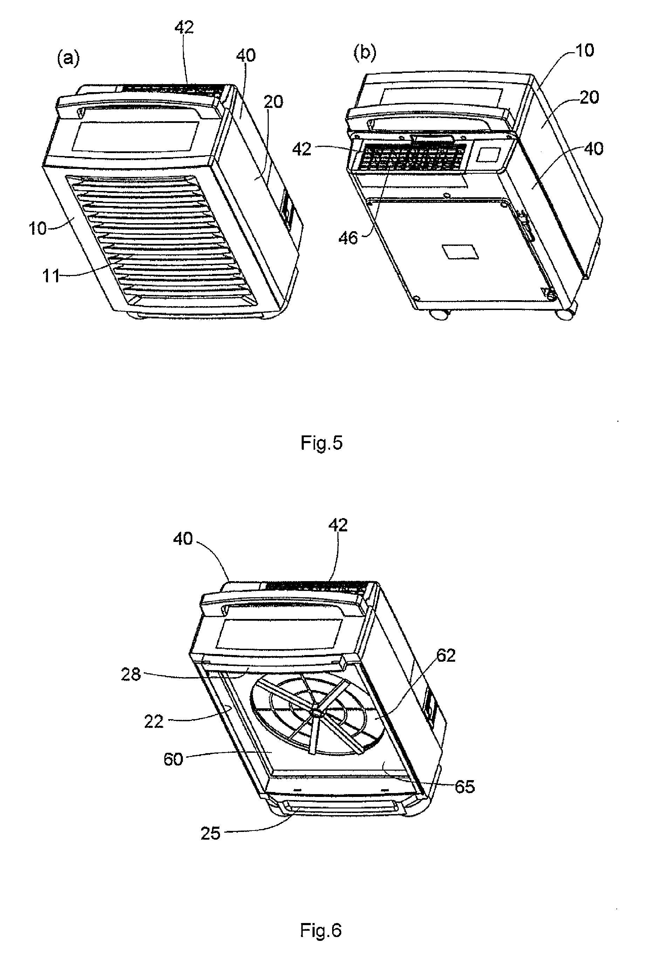

With reference to Figures 1 to 6, an air purifier A according to this embodiment includes a body case C that forms an outer shell, and various functional components such as a deodorizing portion 60 provided in the body case C.

The body case C has a box shape made of resin, and includes various components such as a front panel 10, a front case 20, and a rear case 40.

The front case 20 includes, as a base, a frame 21 having a rectangular shape on front view and a predetermined depth. A rectangular front opening 22 is formed on a front side of the frame 21, and an opening on a rear side is covered with a partition plate 23.

A circular rear opening 24 that opens rearward is formed in the partition plate 23. Specifically, in the front case 20, the front opening 22 and the rear opening 24 communicate with each other.

The rear opening 24 in the partition plate 23 has a circular shape, and a center of the opening is offset to a left from a lateral center when seen from the front of the body. The rear opening 24 opens in a position facing a fan opening 44d of a blowing fan 44 described later, and forms a bell mouth around the fan opening 44d.

Next, a lower side of the frame 21 of the front case 20 entirely protrudes forward beyond left and right sides to form a lower protrusion 25.

An upper side of the frame 21 protrudes forward beyond the left and right sides to form an upper protrusion 28, and an operating portion 26 including a plurality of operation buttons and an LED forming a display portion is provided on an upper front part of the upper side.

Correspondingly to the operating portion 26, an operating board (not shown) on which the operation buttons and the LED are mounted is provided on an upper inner part of the upper side of the frame 21. The operating board is electrically connected to a control portion 47 described later.

Next, the front panel 10 has a rectangular shape on front view, and is configured to cover the front opening 22 of the front case 20 from the front.

Laterally extending slits are formed in a front surface of the front panel 10 to form an air inlet 11 (hereinafter, an inlet 11) that provides communication in a front-rear direction of the front panel 10. Specifically, the front panel 10 ensures ventilation so that air can flow through the front panel 10 in the front-rear direction.

Next, the rear case 40 has a rectangular shape on front view, has a front opening 41 on a front side and an opening to be an air outlet 42 (hereinafter, an outlet 42) in an upper surface, and is formed into a box shape with a closed rear surface 43. The outlet 42 is located on a right side when seen from the front of the body.

On the rear surface 43, a blowing fan 44 as blowing means for taking indoor air into the air purifier, and a partition plate 45 of a scroll shape that forms an air trunk for introducing air flowing down from the blowing fan 44 to the outlet 42 are provided.

Also, in a space formed by the rear case 40 and the partition plate 45 below the partition plate 45, the control portion 47 is provided that controls each component of the air purifier A based on a predetermined program.

Further, a louver 46 that changes a direction of air blown from the outlet 42 into a room or closes the outlet 42 is provided near the outlet 42 in an upper part inside the rear case 40.

A grid is mounted to the opening of the outlet 42 so that the louver 46 is not directly touched.

Next, as the blowing fan 44, a multi-blade fan (sirocco fan) having multiple blades with a predetermined width mounted in a rotational direction is used. Specifically, the blowing fan 44 includes multiple blades 44a located at positions from a predetermined radius from a rotating shaft.

The blowing fan 44 is mounted to the rear surface 43 of the rear case 40 so that a motor 44b that rotationally drives the blades 44a has a rotating shaft 44c directed forward and extending horizontally.

A fan opening 44d surrounded by the blades 44a opens forward.

The blowing fan 44 is thus mounted to the rear case 40, and thus the blowing fan 44 sucks air from the fan opening 44d directed forward in an axial direction of the rotating shaft 44c, and discharges the air radially of the blowing fan 44 including an upper part of the blowing fan 44.

A center of rotation of the blowing fan 44 is offset to a left from a lateral center when seen from the front of the body, and in this state, the fan opening 44d faces the rear opening 24. The blowing fan 44 is configured to rotate to the left when seen from the front of the body and thus suck air.

Next, the partition plate 45 substantially vertically stands on the rear surface 43 of the rear case 40 so as to surround the blowing fan 44, one end thereof is connected to a right end 42a as an opening edge of the outlet 42, and the other end thereof is connected to a left end 42b of the outlet 42.

Specifically, the partition plate 45 surrounds the blowing fan 44, forms a bag shape by the opposite ends being connected to the opening edges of the outlet 42, forms a trunk of air blown out from the blowing fan 44, and is placed in the rear case 40.

For the air trunk formed by the partition plate 45 and leading to the outlet 42, the blowing fan 44 is placed to the left from the lateral center of the body of the air purifier A, and thus a space between a right side of the fan 44 and the partition plate 45 is larger than a space between a left side of the fan 44 and the partition plate 45.

The blowing fan 44 and the partition plate 45 are thus configured, and air blown out from the blowing fan 44 flows along the partition plate 42 to the outlet 42 while rotating to the left with rotation of the blowing fan 44.

At this time, the blowing fan 44 is offset to the left when seen from the front of the body, and a large space is formed between the right side of the blowing fan 44 and the partition plate 45. Thus, air can efficiently flow from the blowing fan 44 to the outlet 42 opening in the right upper part of the body.

Specifically, the flow of air from the blowing fan 44 to the outlet 42 can be efficiently formed within a limited lateral width of the body.

In this embodiment, the center of the blowing fan 44 is offset to the left and the outlet 42 is offset to the right, but the center of the blowing fan may be offset to the right and the outlet 42 may be offset to the left. In this case, a space between the left side of the blowing fan 44 and the partition plate is larger than a space between the right side of the blowing fan 44 and the partition plate, and the blowing fan rotates to the right.

Next, the louver 46 includes a plurality of plate-like wind direction plates 46a, a link mechanism 46c that connects the plurality of wind direction plates 46a and moves the wind direction plates 46a to a predetermined angle, and a drive portion (not shown) such as a motor that drives the link mechanism 46c.

In the louver 46, the plurality of plate-like wind direction plates 46a are arranged in parallel at predetermined intervals on the opening of the outlet 42. Each wind direction plate 46a is journaled on the outlet 42 by shafts 46d formed at opposite ends of the wind direction plate 46a.

A drive portion that drives the link mechanism 46c is connected to the control portion 47 described later, and driven by a predetermined program depending on states of the air purifier A, thereby changing a direction of the louver 46.

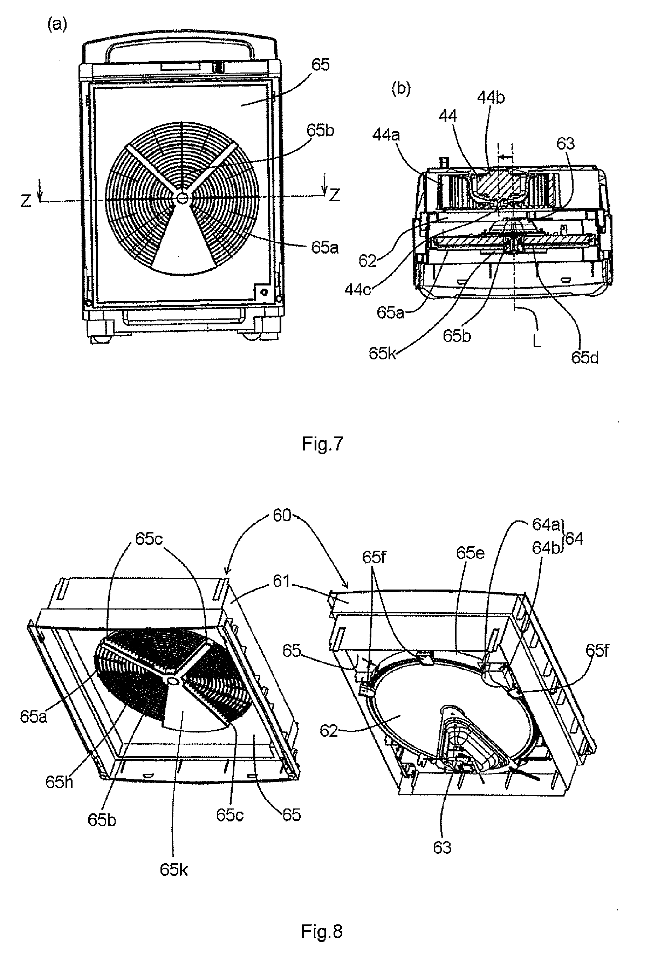

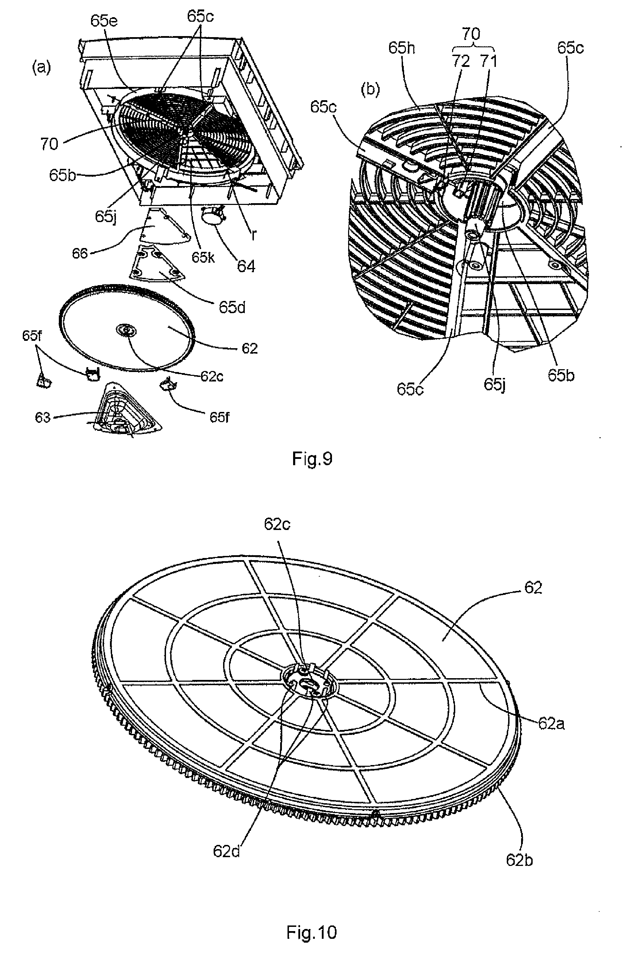

Next, with reference to Figures 8 and 9, a deodorizing portion 60 is a component through which indoor air taken into the air purifier A is passed to remove an odor from the air. The deodorizing portion 60 includes a frame 61 as a base on which various components are provided, a deodorizing element 62, a heating unit 63 that locally heats the deodorizing element 62, and drive means 64 as position changing means for moving the deodorizing element 62 and changing a relative positional relationship between the heating unit 63 and a facing part of the deodorizing element.

First, the frame 61 is made of synthetic resin, has a rectangular shape on front view, and has a predetermined width. An outer shape of the frame 61 is sized to be fitted into the front opening 22 of the front case 20. A resin material of the frame 61 is thermoplastic resin having a heat-resistant temperature of 90°C to 110°C (for example, ABS resin).

As such, a highly heat-resistant component is partially used in the air purifier A, thereby ensuring formability and good appearance design at low cost as compared to use of a frame 61 entirely made of a highly heat-resistant material.

In the frame 61, an intermediate partition plate 65 is provided so as to block an opening of the frame 61 (so as to partition the opening in the front-rear direction).

A circular opening 65 that provides communication in the front-rear direction of the frame 61 is formed in the intermediate partition plate 65. A central support 65b is located at a center of the opening 65a, and a plurality of beams 65c are formed extending radially from the central support 65b to an opening edge of the opening 65a. The beam 65c supports the central support 65b. The central support 65b has a shaft 65j protruding rearward.

With reference to Figure 7, the center of the opening 65a, that is, the central support 65b is located on the lateral center (on a centerline L) when seen from the front of the body.

An optical sensor 70 is provided in a part near the shaft 65j where the central support 65b connects to the beam 65c. The optical sensor 70 includes a light emitting portion 71 and a light receiving portion 72. The light emitting portion 71 and the light receiving portion 72 are placed to face each other with a predetermined space therebetween radially of the opening 65a with reference to the shaft 65j.

The optical sensor 70 is connected to the control portion 47, and when the light receiving portion 72 detects a light emitted from the light emitting portion 71 (ON state), a detection signal is transmitted to the control portion 47.

Further, a frame 65h that allows air to flow into the opening 65a is provided on the front side of the opening 65a. The frame 65h prevents a user from directly touching the deodorizing element 62 describe later.

Further, on a back surface (rear surface) of the intermediate partition plate 65, a ring-like guide portion 65e is formed standing rearward to surround the opening 65a.

A receiving portion 65f that receives the deodorizing element 62 described later when mounted to the guide portion 65e is provided on an edge of the guide portion 65e so as to protrude inward of the opening 65a.

The guide portion 65e is located on an outside of a circular opening edge of the circular opening 65a. Specifically, a gap r is formed between the edge of the opening 65a and the guide portion 65e. A diameter of the ring formed by the guide portion 65e is sized to hold therein the deodorizing element 62 including components described later.

A fan-like region that is located below the central support 65b of the opening 65a in the intermediate partition plate 65, and formed with a predetermined open angle equally extending to the left and right around the central support 65b is covered with a plate fan-like lid 65d.

The lid 65d is made of stainless, and secured to the beam 65c by a screw or the like from the rear side (back side) of the intermediate partition plate 65.

The lid 65d is placed to face the heating unit 63 described later with the deodorizing element 62 therebetween. The lid 65d is sized to cover a heater unit 63a of the heating unit 63 when facing the heater unit 63a.

Specifically, the heating unit 63 and the lid 65d face each other with the deodorizing element 62 therebetween to form a heating space for the deodorizing element 62 described later.

A side of the lid 65d facing the heating unit 63 is painted black to be heat resistant so that heat from the heating unit 63 passing through the deodorizing element to the lid 65d is more strongly transferred to the deodorizing element 62.

With such a configuration, when the lid 65d is heated by the heat from the heating unit 63, the heat is radiated from the surface of the lid 65d painted black toward the deodorizing element 62 to further increase a temperature of the deodorizing element 62. Specifically, the lid 65d is painted black to efficiently generate radiation heat.

Other than black as described above, the surface of the lid 65d may be a gloss surface, and thus the heat from the heating unit 63 passing through the deodorizing element to the lid 65d can be strongly reflected toward the deodorizing element 62.

As such, the lid 65d is formed to have a black surface or a gloss surface, thereby more efficiently increasing a temperature of a catalyst that constitutes the deodorizing element 62.

Further, a plate-like protective member 65k is provided in a part, facing the lid 65d, on the front side of the intermediate partition plate 65. The protective member 65k has a fan shape that can cover the lid 65d, and is made of a highly heat-resistant material (for example, SPS (syndiotactic polystyrene) resin).

As such, the protective member 65k that covers the lid 65d from the front side of the intermediate partition plate 65 is provided to prevent heat deformation of the frame due to heat generation of the heating unit 63. This can eliminate an influence of a rotational operation of the deodorizing element 62 due to heat deformation of the frame.

A heat insulating material 66 is provided between the protective member 65k and the lid 65d. The heat insulating material 66 is thus provided to prevent heat transfer to the protective member 65k due to a temperature increase of the lid 65d, prevent heat radiation from the lid 65d, prevent heat transfer to the frame, and prevent a temperature increase of the frame.

Next, with reference to Figure 10, the deodorizing element 62 has a disk planar shape, and includes a honeycomb core made of ceramic or aluminum, having a plurality of openings like honeycomb openings, and coated or impregnated with a catalyst by a binder.

As the catalyst, a catalyst is used having a property of absorbing an odor (particularly, ammonia odor) such as a catalyst with platinum or manganese.

An opening portion 62c is formed at a center of the deodorizing element 62, and an element frame 62a that is made of stainless and holds the deodorizing element 62 is provided on a front surface directed forward when the deodorizing element 62 is provided in the frame 61.

On the deodorizing element 62, a plurality of angle plates 62d are provided at a predetermined distance from the center of the deodorizing element 62 (center of the opening portion 62c). The angle plate 62d vertically stands on the surface of the deodorizing element 62, and the surface of each angle plate 62d is directed toward the center of the deodorizing element 62.

The angle plates 62d are located at the same distance from the center of the deodorizing element 62, and adjacent angle plates 62d are provided at a predetermined interval. Specifically, the plurality of angle plates 62d are annularly placed around the center of the deodorizing element 62.

In this embodiment, the angle plates 62d are placed adjacent to each other at intervals of an open angle θ of 45° around the deodorizing element 62.

The distance between the center of the deodorizing element 62 and the angle plate 62d is equal to a distance between the center of the central support 65d (shaft 65j) described above and a gap between the light emitting portion 71 and the light receiving portion 72 facing each other.

As described above, the deodorizing element 62 is formed of a honeycomb core, and the element frame 62a provided on the front surface has a predetermined opening. Thus, air can flow through the deodorizing element 62 in the front-rear direction.

Further, a gear portion 62b is provided around the deodorizing element 62 in a peripheral edge of the deodorizing element 62.

A diameter of the deodorizing element 62 including the gear portion 62b is larger than a diameter of the circular opening 65a formed in the intermediate partition plate 65.

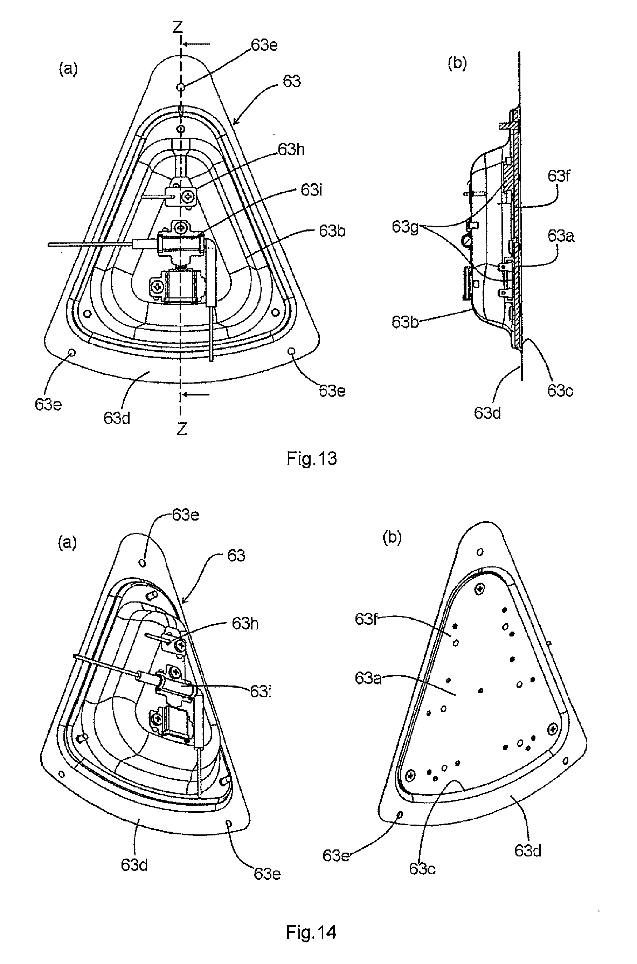

Next, with reference to Figures 13 and 14, the heating unit 63 includes a heater unit 63a as heating means for heating the deodorizing element 62, and a case 63b having a predetermined inner space that houses the heater unit 63a therein.

The heater unit 63a is electrically connected to the control portion 47, and energization is controlled depending on states of the air purifier A.

The heater unit 63a includes a plate-like heat generation portion 63f, and a heater portion 63g that heats the heat generation portion 63f. Further, the heat generation portion 63f has a fan-like planar shape, and a surface thereof is painted (black) to be heat resistant so as to increase emissivity of heat received from the heater portion 63f.

Specifically, in the heater unit 63a, the plate-like heat generation portion 63f receives the heat generated by the heater portion 63g, and the heat generation portion 63f radiates heat from the entire plate surface, thereby heating the facing deodorizing element 62 with reduced irregularities.

When the heater unit 63a is set to a heating capacity such that when the heater unit 63a is energized for a predetermined time, a part facing the deodorizing element 62 placed with a predetermined gap from the heater unit 63a can be heated to a predetermined temperature at which an odor absorbed by the deodorizing element 62 can be removed.

As the heater portion 63g, a PTC heater of semiconductor ceramic mainly consisting of barium titanate is used.

The PTC heater has self-temperature controllability, does not require external temperature control, and does not perform intermittent control as a thermostat. Thus, the PTC heater can be stably used without generation of spark or noise.

Next, the case 63b has a recess 63c that holds the heater unit 63a therein, and a flange portion 63d extending from an opening peripheral edge of the recess 63c.

The recess 63c has a fan shape matching the planar shape of the heater unit 63a, and the heater unit 63a is provided therein so as to face an opening of the recess 63c. The flange portion 63d has a screw hole 63e through which a screw is passed when the heating unit 63 is mounted to a predetermined position.

The heating unit 63 configured as described above has a fan-like planar shape matching the shape of the heat generation portion of the heater unit 63a, and the opening of the recess 63c also has a fan shape.

Next, with reference to Figures 8 and 9, the drive means 64 is position changing means for rotating the deodorizing element 62 and changing a relative positional relationship between the heating unit 63 and the facing part of the deodorizing element, that is, changing the part of the deodorizing element 62 facing the heating unit 63.

The drive means 64 includes a motor 64a, and a bracket 64b that holds the motor 64a. A gear is mounted to a rotating shaft of the motor 64a. The motor 64a is electrically connected to the control portion 47, and energization is controlled depending on the states of the air purifier A.

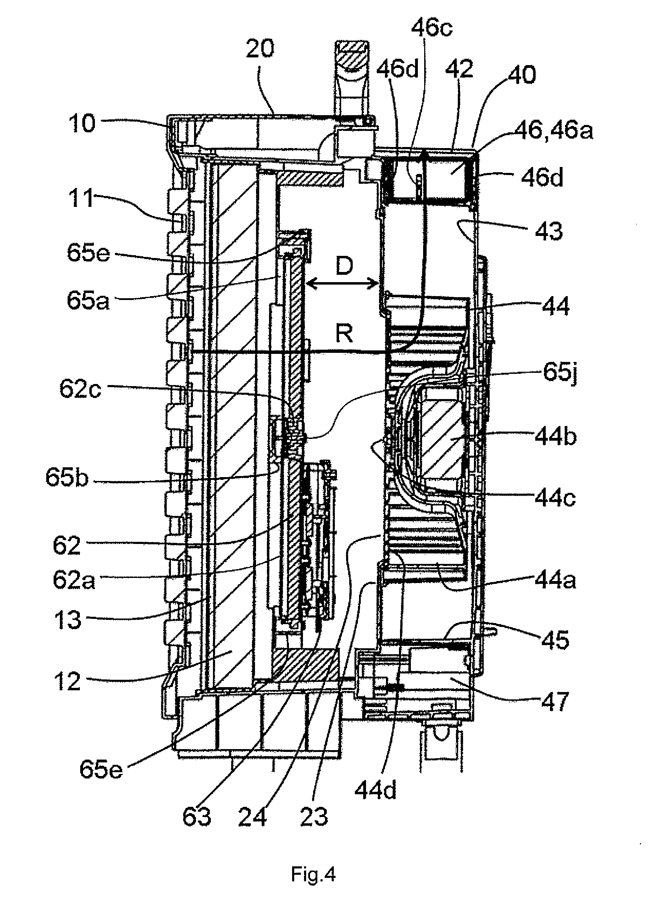

With reference to Figures 4, 7 to 9, the deodorizing element 62, the heating unit 63, and the drive means 64 are mounted to the frame 61 as described below to configure the deodorizing portion 60.

First, the opening portion 62c of the deodorizing element 62 as a bearing rotatably fits the shaft 65j provided on the central support 65b from a back side of the frame 61. Specifically, the central support 65b rotatably supports the deodorizing element 62.

As such, the deodorizing element 62 is mounted to the central support 65b, and thus the center of rotation of the deodorizing element 62 is located at the lateral center when seen from the front of the body.

The rotating shaft 44c as the center of rotation of the blowing fan 44 is offset to the left from the lateral center when seen from the front of the body, and thus the center of rotation of the blowing fan 44 is offset to the left from the center of rotation of the blowing fan 44.

Thus, the deodorizing element 62 is rotatably placed in the frame 61 with the deodorizing element 62 facing the opening 65a inside the guide portion 65e formed on the back (rear) surface of the frame 61.

In this state, the receiving portion 65f that protrudes inward of the opening 65a at the edge of the guide portion 65e and receives the deodorizing element 62 holds the deodorizing element 62 from a rear (back) side so as not to significantly prevent movement of the deodorizing element 62 in a rotational direction.

The deodorizing element 62 is thus mounted to the frame 61, and the deodorizing element 62 is rotatably held to face the opening 65a in the intermediate partition plate 65 in a space surrounded by a back surface of the intermediate partition plate 65 (gap r), the guide portion 65e, and the receiving portion 65f.

The deodorizing element 62 may be held by the guide portion 65e rather than the shaft 65j provided on the central support 65b and the opening portion 62a of the deodorizing element 62 being configured in the relation of the shaft and the bearing as described above.

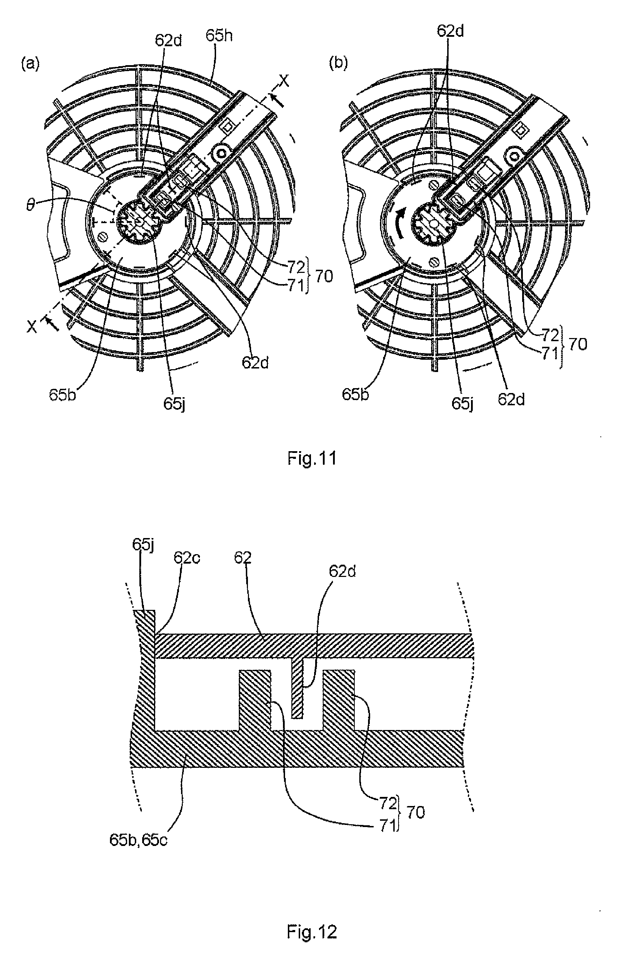

With reference to Figures 11 and 12, with the deodorizing element 62 being held in the frame 61, the distance between the center of the deodorizing element 62 and the angle plate 62d is equal to the distance between the center of the central support 65d (shaft 65j) and the gap formed between the light emitting portion 71 and the light receiving portion 72. Thus, the deodorizing element 62 is mounted to the central support 65d, and the angle plate 62d can be located in the gap formed between the light emitting portion 71 and the light receiving portion 72.

Thus, the angle plate 62d can block direct facing between the light emitting portion 71 and the light receiving portion 72.

Specifically, the deodorizing element 62 is supported by the shaft 65j and rotated, and thus the angle plate 62d passes through the gap between the light emitting portion 71 and the light receiving portion 72. Thus, the angle plate 62d causes the light emitting portion 71 and the light receiving portion 72 to directly face each other (state in Figure 11(b)), or the blocks the facing state (state in Figure 11(a)), and a detection/non-detection state of a light by the light receiving portion 72 can be formed depending on the rotation angle of the deodorizing element 62.

As such, the optical sensor 70 including the light emitting portion 71 and the light receiving portion 72, and the angle plate 62d overlap in the front-rear direction to configure angle detection means for detecting a rotation angle of the deodorizing filter 62.

In this embodiment, the angle plates 62d are placed at predetermined intervals of the open angle θ of 45° from the center of the deodorizing element 62. Thus, for each rotation of 45° of the deodorizing element 62, a light from the light emitting portion 71 to the light receiving portion 72 is blocked.

Specifically, a light detection signal transmitted to the control portion is interrupted for each rotation of 45° of the deodorizing element 62.

Thus, the control portion can properly detect the rotation angle of the deodorizing element 62. Finer setting of the angle allows detection of a finer rotation angle of the deodorizing element 62.

Then, with the deodorizing element 62 being placed on the frame 61 as described above, the heating unit 63 is mounted to the frame 61 so as to partially cover the deodorizing element 62 as described below.

First, the heating unit 63 is placed to span a part from the center to the lower side of the deodorizing element 62 so as not to prevent rotation of the deodorizing element 62.

In this state, the opening of the recess 63c provided with the heater unit 63a is directed forward so that the heater unit 63a of the heating unit 63 directly closely faces the deodorizing element 62.

The heating unit 63 is screwed to the central support 65b located in the opening portion 62c of the deodorizing element 62, and a mounting position formed in the intermediate partition plate 65 located outside the deodorizing element 62.

In this state, the heating unit 63 and the lid 65d face each other via the deodorizing element 62. The heating unit 63 having a fan-like planar shape covers the part from the center of rotation to the lower side of the deodorizing element 62 with the same area on the left and right.

With the configuration as described above, the heating unit 63 is secured to the frame 61 so as not to prevent movement of the deodorizing element 62 in the rotational direction.

The heating unit 63 and the lid 65d face each other and are placed in the frame 61, and thus a space storing heat from the heater unit 63a is formed with the deodorizing element 62 placed between the heating unit 63 and the lid 65d. Further, the heat generation portion 63f is painted to increase heat emissivity, and thus efficiently radiates heat received from the heater portion 63g.

Thus, the heating unit 63 is configured to efficiently locally heat the part of the facing deodorizing element 62.

Next, in the drive means 64, a bracket 64b is secured to the intermediate partition plate 65 with the motor 64a being held by the bracket 64b. At this time, the gear mounted to the rotating shaft of the motor 64a meshes with the gear 62b provided on the deodorizing element 62.

The drive means 64 is placed between the opening 65a and a corner 65g of the intermediate partition plate 65 on the back surface of the intermediate partition plate 65. Further, the drive means 64 is preferably provided in a part between a corner 65g located in an upper part remote from the heating unit 63 among four corners 65g and the opening 65a.

The drive means 64 is thus provided, and energization control by the control portion 47 drives the motor 64a to allow the deodorizing element 62 to be rotated with respect to the frame 61, and allow a part of the deodorizing element 62 facing the heating unit 63 to be changed.

Specifically, a relative positional relationship between the heating unit 63 and the deodorizing element 62 can be changed.

The drive portion 64 is provided between the opening 65a and the corner 65g, and thus a dead space around the opening 65a formed in the rectangular intermediate partition plate 65 can be effectively used. Further, the drive means 64 is provided in a position remote from the heating unit 63, and thus the drive portion 64 is insulated from the influence of heat from the heating unit 63.

Next, the front panel 10, the front case 20, the rear case 40, and the deodorizing portion 60 thus configured are assembled with other functional components as described below to configure the air purifier A. First, the rear case 40 is mounted to the rear surface of the front case 20 with the opening 41 being directed forward. At this time, the opening 44d of the blowing fan 44 provided in the rear case 40 faces the rear opening 24 formed in the partition plate 23 provided in the front case 20. The center of the rear opening 24 matches the axis of the rotating shaft of the blowing fan 44 in the front-rear direction.

Then, the deodorizing portion 60 is mounted to the front case 20 in such a manner that the frame 61 is inserted into the front case 20 from the front opening 22 of the front case 20, and an outer periphery of the frame 61 is held in the front case 20.

As such, with the deodorizing portion 60 being mounted to the front case 20, the rear side of the deodorizing portion 60 (side on which the heating unit 63 is mounted) is placed toward the rear opening 24 of the front case 20, and the heating unit 63 is located between the deodorizing element 62 and the rear opening 24.

The deodorizing element 62 faces the partition plate 23 and the rear opening 24 of the front case 20 that forms a bell-mouth around the opening 44d of the blowing fan 44 with a predetermined space D therebetween so as not to prevent an airflow from the deodorizing element 62 to the rear opening 24.

As such, a distance is ensured between an air sucking portion of the blowing fan 44 and a component compressing an airflow such as the deodorizing element 62, thereby ensuring air sucking efficiency of the blowing fan 44 and preventing a reduction in aerodynamic performance.

The space D is about 40 mm to 60 mm.

The heating unit 63 is located in the space D thus formed.

Then, a HEPA filter 12 of substantially the same size as the opening of the frame 61 is provided in the frame 61 of the deodorizing portion 60 mounted to the front case 20, and a prefilter 13 is provided to cover a front surface of the HEPA filter 12.

Then, the front panel 10 is provided between the upper protrusion 28 and the lower protrusion 25 on the front case 20 on the front side of the prefilter 13 to configure the air purifier A.

The HEPA filter 12 is a filter for removing fine dust such as pollen, mite feces, mold spore, and house dust contained in air.

The prefilter 13 is a coarse filter for previously removing large dust contained in air before the HEPA filter filters the air, and for maintaining an effect of the HEPA filter for a long period.

Next, with reference to Figure 4, in the air purifier A configured as described above, an air trunk R is formed that takes in indoor air, purifies and deodorizes the air, and discharges the air into a room. The air trunk R will be described with an air purifying operation state of the air purifier A and a flow of air taken in.

First, a user operates the operating portion 26 to input to the control portion 47, and thus a predetermined program for operating the air purifier A is executed.

When the blowing fan 44 is driven after the operation start described above, a suction force is generated for taking indoor air from the air inlet 11 into the air purifier A, and the indoor air flows into the air inlet 11.

The air taken in from the air inlet 11 flows rearward in the air purifier A, large dust is removed from the air by the prefilter 13, and then fine dust is removed by the HEPA filter 12.

Then, the air from which dust is removed flows further rearward and reaches the deodorizing portion 60, passes through the opening 65a, and reaches the deodorizing element 62 placed to face the opening 65a. The deodorizing element 62 has many openings in the form of a honeycomb from the front surface to the back surface, and a catalyst that absorbs an odor is applied to the front surface.

Thus, air containing an odor passes through the openings in the form of a honeycomb when passing from the front side to the back side of the deodorizing element 62, and the catalyst applied to the deodorizing element 62 absorbs the odor contained in the air, thereby removing the odor from the air.

"Removing the odor from the air" includes a state where the odor is completely removed from the air, and also a state where the odor is reduced from the state of the air before passing through the deodorizing element 62.

The air purifier A is continuously operated as described above, and thus absorbed odors are accumulated in the deodorizing element 62. With increasing absorbed odors, a deodorizing capacity of the deodorizing element 62 is reduced.

Then, the air from which the dust and the odor are removed flows further rearward from the deodorizing element 62, passes through the rear opening 24 that opens in the partition plate 23 of the front case 20, and flows to the blowing fan 44 placed to face the rear opening 24.

The air flowing to the blowing fan 44 flows from a front side in an axial direction of the blowing fan 44 into the fan opening 44d surrounded by the blade 44a, and is discharged to the outside of the blowing fan 44 radially of the blowing fan 44 including the upper part of the blowing fan 44.

The air discharged from the blowing fan 44 is guided to the rear case 40 by the partition plate 45, adjusted in wind direction when passing through the louver 46, and blown out as purified air through the air outlet 42 upward of the air purifier A from inside the air purifier A.

Thus, the air trunk R horizontally connects to a rear portion of the air purifier body from the air inlet 11, turns upward at the rear portion and reaches the air outlet 42.

Specifically, in the air trunk R, in terms of the airflow, dust filtration filters such as the prefilter 13 and the HEPA filter 12 are placed upstream of the deodorizing element 62, and a bent portion at which the direction of the airflow is changed to be bent upward is formed downstream of the deodorizing element 62. A sirocco fan as the blowing fan 44 is located in the bent portion.

The sirocco fan takes in air from the direction of the rotating shaft of the fan, and discharges the taken air radially of the fan. This forms a linear flow of the indoor air rearward from the front side of the body case C, and can efficiently change the wind direction toward the outlet 42.

The deodorizing element 62, the prefilter 13, the HEPA filter 12, and the front surface of the opening 44d of the blowing fan 44 are placed perpendicularly to the direction of air flowing through the air trunk R.

Thus, the air flows straight to the deodorizing element, and the air hits perpendicularly to each filter surface, thereby providing a good flow of air.

The opening 65a is located at a vertical center on the front side of the body case C, and a relationship between a projected area X of the body case C on front view and an area Y of the opening 65a on front view is "Y ≥ 0.6X."

Next, for the deodorizing element 62 that has absorbed many odors and been reduced in deodorizing performance through an air purifying operation (deodorizing operation) as described above, an operation for recovering the deodorizing performance of the deodorizing element 62 will be described.

The control portion 47 performs an operation for recovering the deodorizing performance as described below at predetermined timing, for example, when a cumulative operation time from an operation start or a previous operation for recovering the deodorizing performance of the deodorizing element 62 exceeds a predetermined time.

In a state where the blowing fan 44 stops, that is, after the air purifier A finishes the air purifying operation, or in a state where the air purifier A does not perform the air purifying operation, the control portion 47 energizes the heater unit 63a to cause the heater unit 63a to generate heat, and heat the part of the deodorizing element 62 facing the heater unit 63a to a predetermined temperature for a predetermined time.

The heating temperature and the heating time of the deodorizing element 62 are sufficient for removing the odor absorbed by the deodorizing element 62.

For the heated part of the deodorizing element 62, the front side is covered with the lid 63d painted to increase heat emissivity, and the rear side is covered with the heating unit 63 (heater unit 63a). Thus, heat generated from the heater unit 63a and heat radiated from the lid 63d can efficiently heat the deodorizing element 62.

The deodorizing element 62 is located between the heater unit 63a and the lid 63d, and thus heat radiated from these members is easily stored near the deodorizing element 62, thereby more efficiently heating the deodorizing element 62.

Next, as described above, when the operation for recovering the deodorizing performance of the part facing the heating unit 63 is finished, the control portion 47 operates the drive means 64 for rotating the deodorizing unit 62 to rotate the deodorizing unit 62 by a predetermined angle.

By this operation, the part of the deodorizing element 62 facing the heating unit 63 and having been heated is offset in the rotational direction with respect to the heating element 63.

For the above described steps, the control portion 47 controls each component using a control program including process steps as described below.

First, in a state where the light from the light emitting portion 71 to the light receiving portion 72 is blocked by the angle plate 62d (in a state where the previous recovery step of the deodorizing element has been finished), the drive means 64 is driven to rotate the deodorizing element 62 in a predetermined direction.

Then, when the control portion 47 detects that the angle plate 62d rotating with the deodorizing element 62 has blocked the light from the light emitting portion 71 to the light receiving portion 72, the control portion 47 stops the drive means 64 to stop rotation of the deodorizing element 62.

Specifically, the deodorizing element 62 is rotationally driven, and the angle plate 62d next to the angle plate 62d that has blocked the light to the light receiving portion 72 in an initial state, on the opposite side in the rotational direction of the deodorizing element 62 blocks the light from the light emitting portion 71 to the light receiving portion 72. Thus, the deodorizing element 62 is rotated by a predetermined angle and stopped.

Next, the control portion 47 energizes the heating unit 63 for a predetermined time with the deodorizing element 62 being stopped to heat the part of the deodorizing element 62 facing the heating unit 63, thereby recovering the deodorizing capacity of the deodorizing element 62.

The above described control program may include a process step of stopping the operation of the drive means and performing a predetermined error display in a case where the light from the light emitting portion is not again blocked by the angle plate even if the drive means as position changing means of the deodorizing element is operated for a predetermined time.

From the above, the part of the deodorizing unit 62 having been heated is moved out of the position between the heating unit 63 and the lid 63d, and a part of the deodorizing element 62 having newly absorbed many odors is located between the heating unit 63 and the lid 63d and heated, thereby recovering the deodorizing capacity.

The open angle θ of rotation of the deodorizing unit 62 is preferably the same as or smaller than the open angle of the heating portion 63f of the fan-like heater unit 63a. Specifically, an angle formed by adjacent angle plates and the center of the deodorizing element 62 is preferably the same as or smaller than the open angle of the heating portion 63f of the fan-like heater unit 63a.

The rotation angle is thus set, and during one turn of the deodorizing unit 62, all parts of the deodorizing unit 62 stay in front of the heating portion 63f and are heated.

The deodorizing unit 62 may be moved immediately after heating, or immediately before a next air purifying operation.

The components of the air purifier A are configured as described above to obtain the advantages as described below.

As in this embodiment, the relative positional relationship between the deodorizing element 62 and the heating unit 63 that locally heats the deodorizing element 62 can be changed to reduce a size of the heating unit 63.

The reduction in size of the heating unit 63 has an advantage that, for example, there is no need to place a large heater that faces the all parts of the deodorizing element 62 to reliably heat the all parts of the deodorizing element 62, thereby simplifying the structure and reducing cost.

The relative positional relationship between the heating unit 63 and the deodorizing element 62 can be changed. Thus, when the all parts of the deodorizing element 62 are to be deodorized, the part of the deodorizing element 62 facing the heating unit 63 may be changed, and the heating unit 63 does not need to cover the all parts of the deodorizing element 62.

Specifically, the part of the deodorizing element 62 facing the heating unit 63 may be always limited, and thus the heating unit 63 minimally prevents the flow of air through the deodorizing element 62. Thus, more air can flow through the deodorizing element 62, and more odors can be removed from the air at a time.

Further, the relative positional relationship between the deodorizing element 62 and the heating unit 63 can be changed, and thus the heating unit 63 can reliably face and heat each part of the deodorizing element 62.

This can reduce irregular heating between the parts of the deodorizing element 62, and efficiently recover the deodorizing capacity of the deodorizing element 62.

In particular, the deodorizing element 62 is formed into a disk shape, and the deodorizing element 62 is rotatably provided with respect to the body with the center of the deodorizing element 62 matching the lateral center of the air purifier body. Thus, the lateral size of the body can be maximally used to maximize the deodorizing element 62, thereby ensuring a larger deodorizing area of the deodorizing element.

Further, the inlet 11 of the air purifier A is formed in the front surface of the body, and the outlet 42 is formed in either a side surface, a top surface, or a back surface of the body.

With such a configuration, the inlet 11 opening wide can easily face a source of an odor, and thus can more quickly suck the odor and remove the odor from the indoor air.

The outlet 11 is formed in either the side surface, the top surface, or the back surface of the body. Thus, purified air hardly flows to the source of the odor and diffusion of the odor can be prevented.

Further, the deodorizing element 62 is rotatably supported in the body case C, and the heating unit 63 is secured to the body case C close to the surface of the deodorizing element 62.

Thus, the heating unit 63 that causes the heater unit 63a to generate heat is secured in the body case C, and there is no need to consider countermeasures against heat in a wide range in the body case C due to routing of a wire for supplying power to generate heat, or a positional change of a hot part in the body case C.

Also, the deodorizing element 62 is rotated to change the part facing the heating unit 63, and thus the deodorizing element 62 may be simply moved in one direction to cause the all parts of the deodorizing element 62 to face the heating unit 63 without irregularities.

Further, the optical sensor 70 including the light emitting portion 71 and the light receiving portion is provided on the central support on a securing side in the air purifier, and the angle plates 62d that block the light from the light emitting portion 71 to the light receiving portion 72 are annularly arranged at predetermined intervals around the center of the deodorizing element rotated by the drive means. Thus, the control portion can detect whether the light from the light receiving portion 72 is detected, and thus can detect a rotation angle of the deodorizing element 62.

Thus, rotation of the deodorizing element 62 can be controlled depending on the size of the heating unit 63 facing the deodorizing element 62. The heating unit 63 can appropriately face the part of the deodorizing element 62 to be heated at timing of heating for recovering the deodorizing capacity of the deodorizing element 62.

In particular, if the angle plates are provided around the center of the deodorizing element at intervals equal to or smaller than the open angle of the heating plate, the deodorizing element can be controlled in rotation at a rotation angle equal to or smaller than the open angle of the heating plate, thereby causing the heating plate to face the deodorizing element without irregularity. Thus, the all parts of the deodorizing element can be continuously heated.

Further, according to the control program used by the control portion 47, first, in the state where the light from the light emitting portion 71 to the light receiving portion 72 is blocked by the angle plate 62d, the drive means 64 is driven to rotate the deodorizing element 62 in the predetermined direction. Then, when the control portion 47 detects that the angle plate 62d that is rotated with the deodorizing element 62 has blocked the light from the light emitting portion 71 to the light receiving portion 72, the control portion stops the drive means 64 to stop rotation of the deodorizing element 62.

Specifically, the deodorizing element 62 is rotationally driven, and the angle plate 62d next to the angle plate 62d that has blocked the light to the light receiving portion 72 in an initial state, on the opposite side in the rotational direction of the deodorizing element 62 blocks the light from the light emitting portion 71 to the light receiving portion 72. Thus, the deodorizing element 62 is rotated by a predetermined angle and stopped.

Next, the control portion 47 energizes the heating unit 63 for a predetermined time with the deodorizing element 62 being stopped to heat the part of the deodorizing element 62 facing the heating unit 63, thereby recovering the deodorizing capacity of the deodorizing element 62.

The heating of the deodorizing element 62 is thus controlled, and thus the part of the deodorizing element 62 to be heated can appropriately face the heating unit to perform heating without any part that is not heated.

The control program may include a process step of stopping the operation of the drive means 64 and performing a predetermined error display in a case where the light from the light emitting portion 71 is not again blocked by the angle plate even if the drive means 64 as position changing means of the deodorizing element 62 is operated for a predetermined time.

With such a control program, the control portion 47 can recognize a case where the deodorizing element 62 is not operated even if the position changing means is operated, and thus can appropriately respond to perform an error display or a stop of the operation, or the like.

Next, the deodorizing element 62 has a disk shape, and thus a rotation region of the deodorizing element 62 can be minimized with respect to an area of the deodorizing element 62 seen in the direction of the rotating shaft.

Specifically, the placement region of the deodorizing element 62 in the body case C can be minimized.

The deodorizing element 62 is rotated to change the surface facing the heating unit 63. Thus, when the deodorizing element 62 has a disk shape, the heater unit 63a of the heating unit 63 having a size in a diametrical direction of the deodorizing element 62 at least equal to or smaller than a rotation radius of the deodorizing element 62 can heat many regions of the deodorizing element 62.

Further, the deodorizing element 62 has a disk shape, and thus has a configuration for achieving the above described advantages. Also, the circular shape can form a maximum region for an opening area of the body case C as the rectangular opening, and thus a larger region allowing deodorization can be formed.

Thus, more air can flow through the deodorizing element 62, thereby increasing an air volume while maintaining the deodorizing capacity.

Next, the surface of the deodorizing element 62 is coated or impregnated with a catalyst having an ammonia decomposition function. Thus, an air purifier can be configured that can quickly deodorize pet odors or odors in nursing care in hospitals, care facilities, and care sites.

In particular, in this embodiment, odors can be removed from more air in a short time, thereby solving a problem of odors in nursing care in a place used by many people such as hospitals or care facilities.

Next, the blowing fan as blowing means is located in the air trunk, the deodorizing element 62 is located upstream of the blowing fan 44 in the air trunk R, and the heating unit 63 is located between the blowing fan 44 and the deodorizing element 62.

With such a configuration, a space provided for reducing compression (loss of an airflow) caused around the deodorizing element 62 and the opening 44d of the blowing fan 44 can be used to place the heating unit 63.

Next, the heating unit 63 includes the case 63b having an open side facing the deodorizing element 62 and having a predetermined inner space, and the electrical heater unit 63a that is located in the inner space of the case 63b and radiates heat through the opening. The heater unit 63a is set to a heating capacity such that when the heater unit 63a is energized for a predetermined time, the facing part of the deodorizing element 62 can be heated to a predetermined temperature. Thus, the odors absorbed by the deodorizing element 62 can be removed.

The case 63b has a fan shape, and thus can cover the deodorizing element 62 in a minimum area. The open angle of the fan shape is set with reference to a rotation angle of one turn of the deodorizing element 62.

Next, the control portion 47 includes the control program for driving the drive means 64 as the position changing means at predetermined timing and rotating the deodorizing element 62.

Thus, when the deodorizing element 62 is heated, the control portion 47 can automatically cause the part of the deodorizing element 62 to be deodorized to face the heating unit 63.

The above described control program has a process step of driving the drive means 64 at predetermined timing, rotating the deodorizing element by a predetermined rotation angle, then stopping the deodorizing element, and energizing the heating unit for a predetermined time in the stop state.

Thus, a series of operations from the rotation to the heating of the deodorizing element 62 can be automatically performed by the control portion 47.

Next, in the air trunk R, the dust filtration filters 12, 13 are placed upstream of the deodorizing element 62, the front panel 10 as a frame having ventilation is removably mounted to the front side of the body case C. With the frame being removed from the body case C, the dust filtration filters can be taken forward of the body case C.

With such a configuration, the dust filtration filter can be removed from the front side of the body case C, thereby increasing a maintenance property of the dust filtration filters to which large dust tends to adhere.

Next, the air trunk R is bent upward on a downstream side of the deodorizing element 62, and the blowing fan 44 is placed in the bent portion. The blowing fan 44 is a multi-blade fan that rotates around the rotating shaft extending horizontally, and also feeds air upward, which is introduced from the front side of the body case C.

The multi-blade fan (sirocco fan) takes in air from the direction of the rotating shaft of the fan, and discharges the taken air radially of the fan. This forms a linear flow of the indoor air rearward from the front side of the body case C, and can efficiently change the wind direction toward the outlet 42.

Next, the heating unit 63 is mounted to the body below the center of rotation of the deodorizing element 62.

As such, the heating unit including the heater unit 63a or the like and having a certain level of weight is placed in a lower position to lower the center of gravity of the air purifier A. Thus, the air purifier A that can be stably placed on a floor surface can be configured.

Besides, the opening 65a is located at the vertical center on the front side of the body case C, and a relationship between a projected area A of the body case C on front view and an area B of the inlet on front view is "B ≥ 0.6A."

This relationship allows the opening 65a and the inlet to take in indoor air to a maximum with respect to the area of the body case C on front view, and the air purifier that can take in and pass more indoor air to the deodorizing element 62 can be configured.

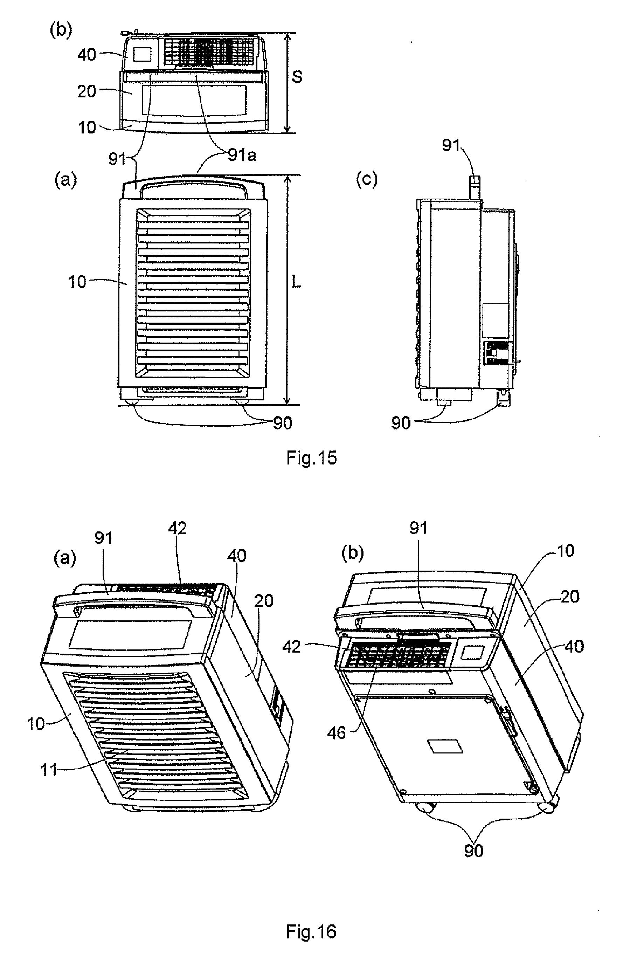

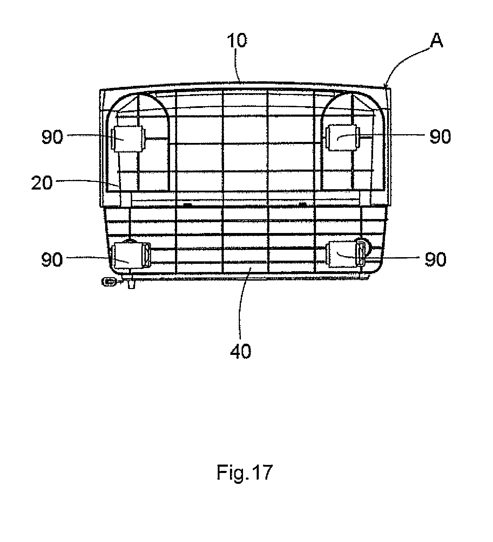

Next, the air purifier A configured as described above is movable with a configuration as shown in Figures 15 to 17.

This will be now described in detail. The air purifier A according to this embodiment includes the body case C as the outer shell and various functional components such as the deodorizing portion 60 provided in the body case C.

The body case C is made of resin, includes multiple components such as the front panel 10, the front case 20, and the rear case 40, and front and side planar shapes form a vertically rectangular outer shell.

Then, four wheels 90 are provided on a bottom of the body case C. Among the four wheels, two are provided on a bottom of the front case 20, and two are provided on a bottom of the rear case 40. When the body case C is seen from the front, one wheel is placed on the left and one wheel is placed on the right so as to be symmetrical with respect to the center of the body case C.

On the upper rear part of the upper side of the frame 21 that constitutes the front case 20, a handle 91 is provided gripped by a user when moving the air purifier A.

The handle 91 is placed so that a central axis of a grip 91a is laterally placed at the center of the top surface of the body case C. The handle 91 is located between the heating unit 63 and the blowing fan 44 in the front-rear direction of the body case C. A height L of the grip 91a from the floor surface is 700 mm or less.

With the components being thus placed, the wheels 90 are configured to be rotatable at least longitudinally of the grip 91a.

As described above, the wheels are provided on the body case C so as to be rotatable longitudinally of the grip 91a of the handle 91, thereby allowing even a large air purifier A to be easily pushed and pulled.

In particular, the handle 91 is located between the heating unit 63 and the blowing fan 44 in the front-rear direction of the body case C, and thus the handle 91 is located substantially between parts with high weight density. Thus, when the handle 91 is gripped to push and pull the air purifier, the air purifier A can be moved at a position having relatively good balance of weight.

This provides the air purifier A easily carried by a user.

The air purifier A of this embodiment is configured so that the height L of the grip 91a of the handle 91 from the floor surface is 700 mm or less.

With such a configuration, the handle 91 can be easily gripped by a user, regardless of gender, without greatly bending down because an average height of dactylus points (vertical distance from a floor surface to a dactylus point of a middle finger when a human stands on the floor surface) of Japanese adults is about 662 mm as a whole (male: about 695 mm, female: about 632 mm).

Further, a depth S of the air purifier A of this embodiment is about 300 mm.

With such a configuration, when the air purifier A is laterally pushed and pulled, the air purifier A is within a shoulder width of a human regardless of gender because an average shoulder width of Japanese adjusts is about 409 mm as a whole (male: about 426 mm, female: about 394 mm).

Thus, the air purifier A can be easily moved even in a narrow passage or place.

Further, a width of a corridor in ordinary houses is about 850 mm to 900 mm, and thus when the air purifier A is transversely placed on a corridor, a space of about 550 mm to 600 mm can be ensured.

Thus, even if the air purifier A is placed on a corridor in ordinary houses, a sufficient space can be ensured through which a human or an object can pass.

Further, a width of a corridor in care facilities is about 1800 mm or more, and thus when the air purifier A is transversely placed on a corridor, a space of about 1500 mm can be ensured.

Thus, even if the air purifier A is placed on a corridor in care facilities, a sufficient space can be ensured through which a human or an object can pass.

Also, a width of a generally used wheelchair is about 700 mm, and thus even if the air purifier A is placed on a corridor in care facilities, wheelchairs can pass each other.

### Description of Reference Characters

- A: air purifier
- C: body case
- R: air trunk
- 10: front panel
- 11: air inlet
- 12: HEPA filter
- 13: prefilter
- 20: front case
- 21: frame
- 23: partition plate
- 26: operating portion
- 40: rear case
- 42: air outlet
- 44: blowing fan
- 45: partition plate
- 46: louver
- 46a: wind direction plate
- 46c: link mechanism
- 47: control portion
- 60: deodorizing portion
- 61: frame
- 62: deodorizing element
- 63: heating unit
- 64: drive means
- 65: intermediate partition plate
- 65d: lid
- 66: heat insulating material
- 67: cover
- 90: wheel
- 91: handle
- 91a: grip

## Claims

1. An air purifier comprising:
a body case having an inlet and an outlet opening outward, and having an air trunk that provides communication between the inlet and the outlet;
blowing means that is included in the body case, and introduces indoor air through the air trunk from the inlet to the outlet;
a deodorizing element that is provided in the air trunk, and through which the introduced air can pass;
a heating unit that locally heats the deodorizing element;
a frame made of synthetic resin and including the deodorizing element and the heating unit;
position changing means for changing a relative positional relationship between the heating unit and the deodorizing element; and
control means for controlling energization of the blowing means, the heating unit, and the position changing means,
wherein the deodorizing element is heated by the heating unit to recover a deodorizing function, and is rotatably mounted to the frame,
the heating unit is secured to the frame while facing the deodorizing element, and
a lid covering the heating unit is provided on a facing side of the heating unit via the deodorizing element.

2. The air purifier according to claim 1, wherein the lid is painted to increase heat emissivity.

3. The air purifier according to claim 1 or 2, wherein a protective member made of a highly heat-resistant material is provided on a part of the frame where the lid is placed.

4. The air purifier according to claim 3, wherein a heat insulating material is provided between the lid and the protective member.

5. The air purifier according to any one of claims 1 to 4, wherein the synthetic resin material of the frame is thermoplastic resin having a heat-resistant temperature of 90°C to 110°C.
